# FASCICULE DE BREVET EUROPEEN

(11) **EP 0 764 465 B1**
(45) Date de publication et mention de la délivrance du brevet: **01.12.1999**
(21) Numéro de dépôt: 96401971.5
(22) Date de dépôt: 16.09.1996
(51) Int. Cl.: B01J 23/89, C10G 2/00, C07C 1/04

(54) **Procédé de conversion du gaz de synthèse en présence d'un catalyseur comprenant du cobalt et des éléments additionnels**
Verfahren zur Konversion von Synthesegas mit einem Katalysator der Kobalt und additionelle Elemente enthält
Process for the conversion of synthesis gas with a catalyst containing cobalt and additional elements

(30) Priorité: 25.09.1995 FR 9511296
(43) Date de publication de la demande: 26.03.1997
(73) Titulaire: INSTITUT FRANCAIS DU PETROLE, 92502 Rueil-Malmaison (FR)
(72) Inventeur: Chaumette, Patrick, 78380 Bougival (FR); Didillon, Blaise, 92500 Rueil Malmaison (FR)

(56) Documents cités:
- WO-A-86/00296
- WO-A-92/06784
- DE-A- 4 219 690
- US-A- 3 099 618
- US-A- 5 086 027

## Description

La présente invention concerne une formulation catalytique, sa préparation et son utilisation dans un procédé de synthèse d'hydrocarbures à partir d'un mélange CO-(CO₂)-H₂, c'est-à-dire d'un mélange CO-H₂ comprenant éventuellement du CO₂ appelé gaz de synthèse, plus particulièrement l'utilisation permettant de réaliser la conversion du gaz de synthèse en un mélange d'hydrocarbures linéaires et saturés essentiellement constitué d'hydrocarbures C₅⁺ (c'est-à-dire possédant au moins 5 atomes de carbone par molécule), ou plus précisement en un mélange d'hydrocarbures essentiellement linéaires et saturés, contenant au moins 80 % poids d'hydrocarbures C₅⁺ par rapport à l'ensemble des hydrocarbures formés.

Il est connu de l'homme du métier que le gaz de synthèse peut être converti en hydrocarbures en présence de catalyseurs contenant des métaux de transition. Cette conversion, opérée à haute température et sous pression, est connue dans la littérature sous le nom de synthèse FISCHER- TROPSCH. Ainsi des métaux du groupe VIII de la classification périodique des éléments tel que le fer, le ruthénium, le cobalt et le nickel catalysent la transformation de mélanges CO-(CO₂)-H₂ en hydrocarbures liquides et/ou gazeux.

Les produits préparés par synthèse FISCHER- TROPSCH en présence de catalyseurs comprenant des métaux du groupe VIII présentent une distribution très large en terme de poids moléculaire. Ainsi seulement une faible proportion des produits obtenus se situent dans la gamme des distillats moyens constitués par des fractions kérosène et gasoil, la (ou les) fraction(s) kérosène étant constituée(s) par un mélange d'hydrocarbures dont les points d'ébullition sont compris entre 140° et 300°C, et la (ou les) fraction(s) gasoil étant constituée(s) par un mélange d'hydrocarbures de points d'ébullition compris entre 180° et 370°C lors d'une distillation atmosphérique telle que réalisée par l'homme du métier sur un brut pétrolier.

Des efforts importants ont été entrepris depuis 1973 afin d'améliorer le rendement en distillats moyens des procédés basés sur la conversion du gaz de synthèse. Le catalyseur décrit dans le brevet US-A-5.302.622, comprenant du cobalt, du cuivre et du ruthénium et préparé par gélification, conduit à l'obtention d'un mélange d'hydrocarbures essentiellement linéaires et saturés contenant au moins 80% poids d'hydrocarbures C₅⁺ par rapport à l'ensemble des hydrocarbures formés.

D'autre part, le catalyseur décrit dans le brevet français FR-A-2.677.992 contient du cobalt, au moins un élément M additionnel (par exemple sous forme métallique ou sous forme d'oxyde) choisi dans le groupe constitué par le molybdène et le tungstène et au moins un élément N additionnel (par exemple sous forme métallique ou sous forme d'oxyde) choisi dans le groupe constitué par les éléments des groupes Ia, IIa, Ib (tels que par exemple le sodium, le potassium, le magnésium, le calcium, le cuivre ou l'argent), le ruthénium, le palladium, l'uranium, le praséodyme et le néodyme, de préférence dans le groupe formé par le sodium, le potassium, le ruthénium, le cuivre et l'uranium, l'ensemble de ces éléments étant dispersés sur un support, qui est de préférence constitué par au moins un oxyde d'au moins un élément choisi dans le groupe formé par les éléments suivants: Si, Al, Ti, Zr, Sn, Zn, Mg, Ln (où Ln est une terre rare).

Les teneurs en éléments dudit catalyseur après calcination, exprimées en poids d'élément par rapport au poids de support, sont habituellement les suivantes:
. 1 à 60%, et préférentiellement de 5 à 40%, en poids de cobalt.
. 0,1 à 60% , et préférentiellement de 1 à 30%, en poids d'élément M.
. de 0,01 à 15% et préférentiellement de 0,05 à 5%, en poids d'élément N.

Le cobalt et les éléments additionnels peuvent être introduits en utilisant toute méthode connue de l'homme du métier telle que, par exemple, l'échange ionique, l'imprégnation à sec, la coprécipitation, la gélification, le mélange mécanique ou le greffage de complexes organométalliques, les techniques d'imprégnation ou de gélification étant préférées.

Le catalyseur selon le brevet français FR-A-2.677.992, après réduction sous hydrogène, permet de convertir le gaz de synthèse en un mélange d'hydrocarbures essentiellement linéaires et saturés contenant au moins 80% poids d'hydrocarbures C₅⁺ par rapport à l'ensemble des hydrocarbures formés. L'emploi des techniques d'imprégnation ou de gélification du cobalt et du molybdène et/ou du tungstène et d'au moins un élément additionnel N et éventuellement au moins un élément choisi dans le groupe formé par les éléments du support, permet d'obtenir un catalyseur de conversion du gaz de synthèse en hydrocarbures à la fois stable, actif et sélectif en hydrocarbures C₅⁺.

La présente invention présente la préparation d'un catalyseur, dont les performances sont stables, et qui conduit après réduction sous hydrogène à la conversion du gaz de synthèse en un mélange d'hydrocarbures essentiellement linéaires et saturés contenant au moins 80% poids d'hydrocarbures C₅⁺ par rapport à l'ensemble des hydrocarbures formés. En particulier, un des avantages dudit catalyseur, utilisé dans un procédé de synthèse Fischer-Tropsch, est l'obtention d'une plus faible quantité de méthane (produit secondaire de ladite réaction de synthèse, non désiré) que lors de l'utilisation des catalyseurs décrits dans l'art antérieur, et particulièrement que par rapport à l'utilisation du catalyseur décrit dans le brevet français FR-A-2.677.992.

La présente invention concerne un procédé de préparation d'un catalyseur comprenant un support choisi dans le groupe formé par au moins un oxyde d'élément choisi dans le groupe formé par les éléments suivants: Si, Al, Ti, Zr, Sn, Zn, Mg, Ln (où Ln est une terre rare, c'est-à-dire un élément de numéro atomique compris entre 57 et 71 inclus), de préférence choisi dans le groupe formé par la silice, l'alumine, la zircone et l'oxyde de titane, et, en % poids d'élément par rapport au poids de support dans le catalyseur, entre 1 et 60 %, de préférence entre 2 et 50 %, de cobalt, entre 0,01 et 20 %, de préférence entre 0,05 et 10 %, d'au moins un élément additionnel A choisi dans le groupe formé par le ruthénium, le platine, le palladium et l'uranium, entre 0,01 et 20 %, de préférence entre 0,02 et 15 %, d'au moins un élément additionnel B choisi dans le groupe formé par le molybdène et le tungstène, ledit procédé étant caractérisé en ce que la préparation du catalyseur comprend au moins les étapes successives suivantes :
- (1) la formation d'un précurseur comprenant au moins le cobalt et au moins une partie, de préférence la totalité, du support
- (2) la réduction au moins partielle dudit précurseur en présence d'au moins un composé réducteur, et
- (3) le dépôt sur le précurseur réduit de toute partie de composé présent dans le catalyseur et non présent dans le précurseur.

L'invention concerne aussi le catalyseur susceptible d'être obtenu par le procédé de préparation selon l'invention, ainsi qu'un procédé de synthèse d'hydrocarbures essentiellement linéaires et saturés, contenant au moins 80 % poids d'hydrocarbures C₅⁺ par rapport à l'ensemble des hydrocarbures formés, à partir d'une charge comprenant du monoxyde de carbone CO, de l'hydrogène et éventuellement du bioxyde de carbone CO₂, ladite charge étant appelée gaz de synthèse.

De façon préférée, le rapport molaire B/Co est généralement inférieur à 1, et de manière encore plus préférée, ledit rapport est généralement compris entre 0,01 et 0,9.

Un des modes préférés de réalisation du procédé selon l'invention est tel que le précurseur comprend en outre une partie de l'élément A présent dans le catalyseur, l'autre partie de l'élément A présent dans le catalyseur étant déposée sur le précurseur réduit à l'étape (3).

Un autre des modes préférés de réalisation du procédé selon l'invention, indépendamment ou non du mode de réalisation précédent, est tel que le précurseur comprend en outre la totalité de l'élément A présent dans le catalyseur. Dans ce cas, au moins une partie de l'élément B, de préférence la totalité de l'élément B, est déposée sur le précurseur réduit à l'étape (3).

Un autre des modes préférés de réalisation du procédé selon l'invention, indépendamment ou non des modes de réalisation précédents, est tel que le précurseur comprend en outre une partie de l'élément B présent dans le catalyseur, l'autre partie de l'élément B présent dans le catalyseur étant déposée sur le précurseur réduit à l'étape (3).

Ainsi un des modes préférés de réalisation du procédé selon l'invention, indépendamment ou non des modes de réalisation précédents, est tel que le précurseur comprend à la fois une partie de l'élément A présent dans le catalyseur et une partie de l'élément B présent dans le catalyseur, les autres parties des éléments A et B étant déposées sur le précurseur réduit à l'étape (3).

Un des modes préférés de réalisation du procédé selon l'invention, indépendamment ou non des modes de réalisation précédents, est tel que le précurseur comprend en outre la totalité de l'élément B présent dans le catalyseur. Dans ce cas, au moins une partie de l'élément A, de préférence la totalité de l'élément A, est déposée sur le précurseur réduit à l'étape (3).

Le cobalt et les éléments additionnels A et B éventuellement présents dans le précurseur peuvent être introduits, à l'étape (1), par toute méthode connue de l'homme du métier, telle que par exemple l'échange mécanique, l'imprégnation à sec, la coprécipitation, la gélification, le mélange ionique ou le greffage de complexes organométalliques. L'étape (1) permet éventuellement d'incorporer dans le précurseur du catalyseur utilisé selon l'invention au moins une partie de l'élément A et/ou une partie de l'élément B, mais en aucun cas l'étape (1) ne permet d'incorporer dans ledit précurseur à la fois la totalité de l'élément A présent dans le catalyseur et la totalité de l'élément B présent dans le catalyseur.

Parmi ces méthodes, les méthodes d'imprégnation ou de gélification sont préférées pour la préparation dudit précurseur à l'étape (1), car elles permettent un contact intime entre le cobalt et le ou les éventuels éléments additionnels A ou B.

En effet, l'emploi des techniques d'imprégnation ou de gélification du cobalt et éventuellement de l'élément A et éventuellement de l'élément B et éventuellement d'au moins un élément choisi dans le groupe formé par les éléments du support permet généralement d'obtenir un précurseur de catalyseur de conversion du gaz de synthèse en hydrocarbures à la fois stable, actif et sélectif en hydrocarbures C₅⁺, et très faiblement sélectif en méthane.

Une méthode préférée de préparation du précurseur du catalyseur utilisé selon l'invention consiste par exemple à imprégner au moins une partie, de préférence la totalité, du support au moyen d'au moins une solution aqueuse (ou dans au moins un solvant approprié) contenant le cobalt, et éventuellement tout ou partie du ou des éléments additionnels A ou B et éventuellement d'au moins un élément choisi dans le groupe formé par les éléments du support, sous forme par exemple d'un halogénure, d'un nitrate, d'un acétate, d'un oxalate, d'un sulfate, d'un complexe formé avec l'acide oxalique et les oxalates, d'un complexe formé avec l'acide citrique et les citrates, d'un complexe formé avec l'acide tartrique et les tartrates, d'un complexe formé avec un autre polyacide ou acide alcool et ses sels, d'un complexe formé avec les acétyl acétonates, et tout autre dérivé inorganique ou organométallique contenant le cobalt et éventuellement tout ou partie du ou des éléments additionnels A ou B, l'autre partie éventuelle du ou des éléments additionnels A ou B et d'élément choisi dans le groupe formé par les éléments du support étant imprégnée par la suite à l'étape (3).

Pour incorporer éventuellement au moins une partie de l'élément B (Mo ou W), il est également possible d'utiliser au moins un molybdate ou au moins un tungstate d'ammonium, tels que le molybdate d'ammonium, l'heptamolybdate d'ammonium tetrahydraté ou le metatungstate d'ammonium par exemple.

Après chaque imprégnation du cobalt et éventuellement du ou des éléments additionnels A ou B et d'élément choisi dans le groupe formé par les éléments du support sur le support choisi, le précurseur obtenu est ensuite traité thermiquement, c'est-à-dire séché, par tout moyen connu de l'homme du métier, par exemple sous un courant d'azote ou d'air à une température comprise entre 80°C et 200°C, puis calciné par exemple sous un courant d'air ou d'azote à une température comprise par exemple entre 200°C et 800°C.

Il est également possible de préparer le précurseur du catalyseur selon l'invention au moyen de la méthode décrite de façon détaillée dans le brevet US-A- 3 975 302 et qui consiste en la préparation d'une solution d'imprégnation à partir d'un gel solide amorphe et d'alkanolamine, puis à imprégner un support avec cette solution.

Une autre méthode de préparation du précurseur de catalyseur selon l'invention, préférée dans le cadre de la présente invention, consiste en la préparation d'un gel contenant le cobalt et le ou les éventuels éléments A ou B et éventuellement au moins un élément choisi dans le groupe formé par les éléments du support. Cette préparation par gélification se fait selon toute technique connue de l'homme du métier. Cependant, deux méthodes de préparation par gélification sont préférées et décrites ci-après, la première méthode ne concernant que la présence de l'élément B dans le précurseur.

La première des méthodes préférées de gélification consiste en la préparation d'un gel contenant le cobalt et éventuellement l'élément B selon la technique décrite dans le brevet US-A-3 846 341 en substituant le sel de fer tel que décrit dans ledit brevet par un sel de cobalt. Ainsi le gel contenant du cobalt, l'éventuel élément B et au moins une partie, de préférence la totalité, du support peut être préparée de la façon décrite ci-après.

Une solution aqueuse I de sel d'élément B, par exemple de paramolybdate d'ammonium si B est Mo, présentant une concentration comprise entre 1 et 2,5 atom.g de molybdène par litre, est introduite dans un réacteur et agitée à une température inférieure à 20°C. A cette solution I est ajoutée une solution aqueuse II contenant un sel de cobalt, préférentiellement du nitrate de cobalt, à une concentration supérieure à 1 atom.g par litre. Une suspension colloïdale est alors obtenue. Cette suspension peut éventuellement être durcie lors d'un réchauffement lent sous agitation faible (v < 1000 rpm). Un vieillissement effectué à une température supérieure à 10°C résulte en l'obtention d'un gel homogène. Si nécessaire, le gel peut être déshydraté à une température comprise entre 40 et 150°C, de préférence entre 50 et 90°C. Il est ensuite séché, par tout moyen connu de l'homme du métier, par exemple sous un courant d'azote ou d'air, à une température comprise entre 80 et 200°C, puis calciné, par exemple sous un courant d'azote ou d'air à une température comprise par exemple entre 200 et 800°C. Le support, en partie ou en totalité, peut être introduit à toute étape de la préparation telle que décrite ci-dessus ; il est de préférence introduit dans la solution I ou dans la solution II, préférentiellement sous une forme finement divisée, c'est-à-dire telle que les grains aient de préférence une taille inférieure à 250 µm (1µm = 1 micron = 1 micromètre = 1,10⁻⁶m).

La seconde des méthodes préférées de gélification est décrite ci-après.

Elle consiste en la préparation d'un gel obtenu en mélangeant une solution I contenant un composé organométallique, de préférence un alcoxyde de l'élément précurseur du support, dissous dans un solvant organique, de préférence un alcool, et une solution aqueuse II contenant un sel de cobalt, éventuellement au moins un sel d'élément A et éventuellement au moins un sel d'élément B et éventuellement au moins un élément choisi dans le groupe formé par les éléments du support et contenant également un acide minéral qui accélère la gélification tel que par exemple l'acide nitrique, l'acide chlorhydrique, l'acide sulfurique ou l'acide phospohrique. Lesdits sels de cobalt, et du ou des éléments éventuels A ou B ou d'élément éventuel choisi dans le groupe formé par les éléments du support, sont par exemple des halogénures, des nitrates, des acétates, des oxalates, des sulfates, des complexes formés avec un polyacide ou un acide alcool et ses sels ou des complexes formés avec les acétylacétonates, ou tout autre dérivé inorganique soluble en solution aqueuse. Le mélange des solutions I et II sous agitation en présence dudit acide conduit à l'obtention d'un gel qui est formé en moins de 10 mn et à une température comprise entre 20 et 80°C. Le gel ainsi formé est séparé des solvants résiduels par tout moyen connu de l'homme du métier, par exemple par centrifugation ou filtration, puis séché, par exemple sous un courant d'azote ou d'air à une température comprise entre 80 et 200°C, et enfin calciné, par exemple sous un courant d'air ou d'azote à une température comprise entre 200 et 800°C.

L'étape (2) de réduction du précurseur formé à l'étape (1) est généralement telle que décrite ci-après.

Le précurseur de catalyseur est tout d'abord préréduit par au moins un composé réducteur, par exemple choisi dans le groupe formé par l'hydrogène, le monoxyde de carbone et l'acide formique, éventuellement mis en contact avec un gaz inerte (azote par exemple) dans un rapport molaire composé réducteur / (composé réducteur + gaz inerte) compris entre 0,001:1 à 1:1.

La réduction est menée en phase gazeuse entre 150 et 600°C, de préférence entre 200 et 500°C, entre 0,1 et 10 MPa et à une vitesse volumétrique horaire comprise entre 100 et 40 000 volumes de mélange par volume de catalyseur et par heure. Cette préréduction peut également être menée en phase liquide, le catalyseur étant mis en suspension dans un solvant inerte, par exemple une coupe paraffinique.

L'étape (3) de dépôt sur le précurseur réduit de toute partie de composé présent dans le catalyseur et non présent dans le précurseur est mise en oeuvre selon toute technique connue de l'homme du métier. Par composé, on entend généralement : élément choisi dans le groupe formé par les éléments du support ou élément A ou élément B, à l'exclusion du cobalt. Une méthode préférée consiste par exemple à imprégner le précurseur réduit au moyen d'au moins une solution aqueuse (ou dans au moins un solvant approprié) contenant éventuellement tout élément choisi dans le groupe formé par les éléments du support et tout ou partie du ou des éléments additionnels A ou B, sous forme par exemple d'un halogènure, d'un nitrate, d'un acétate, d'un oxalate, d'un sulfate, d'un complexe formé avec l'acide oxalique et les oxalates, d'un complexe formé avec l'acide citrique et les citrates, d'un complexe formé avec l'acide tartrique et les tartrates, d'un complexe formé avec un autre polyacide ou acide alcool et ses sels, d'un complexe formé avec les acétyl acétonates, et tout autre dérivé inorganique ou organométallique contenant tout ou partie du ou des éléments additionnels A ou B, l'autre partie éventuelle du ou des éléments additionnels A ou B ayant été imprégnée précédemment à l'étape (1).

Pour incorporer éventuellement au moins une partie de l'élément B (Mo ou W), il est également possible d'utiliser au moins un molybdate ou au moins un tungstate d'ammonium, tels que le molybdate d'ammonium, l'heptamolybdate d'ammonium tetrahydraté ou le metatungstate d'ammonium par exemple.

Après chaque imprégnation du ou des éléments additionnels A ou B, et éventuellement d'élément choisi dans le groupe formé par les éléments du support, le catalyseur obtenu est ensuite séché, par tout moyen connu de l'homme du métier, par exemple sous un courant d'azote ou d'air à une température comprise entre 80 et 200°C. Après ce séchage, le catalyseur peut éventuellement être calciné par exemple sous un courant d'air ou d'azote à une température comprise par exemple entre 200 et 800°C.

Il est également possible de préparer à l'étape (3) le catalyseur selon l'invention au moyen de la méthode décrite de façon détaillée dans le brevet US-A-3 975 302 et qui consiste en la préparation d'une solution d'imprégnation à partir d'un gel solide amorphe et d'alkanolamine, puis à imprégner le précurseur formé à l'étape (1) avec cette solution.

Le catalyseur est éventuellement mis en forme par tout procédé connu de l'homme du métier, par exemple par extrusion, coagulation en goutte, dragéification ou pastillage. Après cette étape de mise en forme, ledit catalyseur subira éventuellement un séchage dans les conditions opératoires précitées.

Les catalyseurs selon l'invention se distinguent par rapport aux catalyseurs de l'art antérieur (brevet français FR-A-2.677.992 notamment) par le fait qu'une proportion plus importante des éléments A et/ou B est déposée sur le cobalt et non sur le support. Ceci peut être visualisé par exemple au moyen d'une analyse par microscopie électronique et détermination des compositions locales par émission X.

Les catalyseurs préparés selon les modes opératoires décrits ci-dessus sont particulièrement bien adaptés pour être utilisés dans les procédés de fabrication d'un mélange d'hydrocarbures essentiellement linéaires et saturés, contenant au moins 80 % en poids d'hydrocarbures C₅⁺ par rapport à l'ensemble des hydrocarbures formés, à partir d'un gaz de synthèse.

Les conditions de mise en oeuvre desdits catalyseurs pour la fabrication d'hydrocarbures selon l'invention sont habituellement les suivantes:

Le catalyseur, chargé dans un réacteur, est soumis à une préréduction avant utilisation, par au moins un composé réducteur, par exemple choisi dans le groupe formé par l'hydrogène, le monoxyde de carbone et l'acide formique, éventuellement mis en contact avec un gaz inerte (azote par exemple) en rapport molaire composé réducteur/(composé réducteur + gaz inerte) compris entre 0,001:1 et 1:1.

La préréduction est menée entre 150 et 600°C, de préférence entre 200 et 500°C, entre 0,1 et 10 MPa et à une vitesse volumétrique horaire comprise entre 100 et 40 000 volumes de mélange par volume de catalyseur et par heure. Cette préréduction peut éventuellement être menée en phase liquide comprenant au moins un hydrocarbure ayant au moins 5, de préférence au moins 10, atomes de carbone par molécule si, par la suite, la réaction de synthèse d'hydrocarbures se déroule en phase liquide comprenant au moins un hydrocarbure ayant au moins 5, de préférence au moins 10, atomes de carbone par molécule.

La conversion du gaz de synthèse en hydrocarbures est ensuite opérée sous une pression totale habituellement comprise entre 0,1 et 15 MPa, et de préférence entre 1 et 10 MPa, la température étant généralement comprise entre 150 et 350°C, et de préférence entre 170 et 300°C.

La vitesse volumétrique horaire est habituellement comprise entre 100 et 20000 volumes de gaz de synthèse par volume de catalyseur et par heure et de préférence entre 400 et 5 000 volumes de gaz de synthèse par volume de catalyseur et par heure, et le rapport H₂/CO dans le gaz de synthèse est habituellement compris entre 1:2 et 5:1, de préférence entre 1,2:1 et 2,5:1.

Le catalyseur est généralement utilisé en poudre fine calibrée (10-700 µm environ) ou en particules de diamètre équivalent compris entre 2 et 10 mm environ, respectivement en présence d'une phase liquide (dans les conditions opératoires) et d'une phase gazeuse, ou d'une phase gazeuse. La phase liquide peut être constituée par au moins un hydrocarbure ayant au moins 5, de préférence au moins 10, atomes de carbone par molécule.

Les catalyseurs selon l'invention sont particulièrement actifs et stables dans la réaction de synthèse d'hydrocarbures à partir de gaz de synthèse. Ils permettent d'obtenir très peu de méthane, produit secondaire non désiré. Finalement, lesdits catalyseurs permettent d'obtenir des hydrocarbures essentiellement paraffiniques, dont la fraction présentant les points d'ébullition les plus élevés peut être convertie avec un rendement élevé en distillats moyens (coupes gasoil et kérosène) par un procédé d'hydroconversion tel que l'hydrocraquage et/ou l'hydroisomérisation catalytiques.

Les exemples qui suivent illustrent l'invention.

### Exemple 1 (selon l'invention): catalyseur E

Une solution 1 contenant 130 g d'orthosilicate d'éthyle (T.E.O.S.) dissous dans 50 ml d'éthanol et une solution 2 contenant 46,3 g de nitrate de cobalt hexahydraté, 0,21 g de trichlorure de ruthénium héxamine et 32 g d'acide nitrique concentré dissous dans 80 cm3 d'eau, sont mélangées sous forte agitation à température ambiante.

L'hydrolyse du T.E.O.S. conduit au bout de 15 mn à la formation d'un gel massique contenant les sels de cobalt et de ruthénium.

Le gel obtenu est séparé des eaux mères, séché à l'étuve entre 40°C et 120°C, puis calciné à 600°C sous air.

Le précurseur de catalyseur ainsi obtenu est réduit, à pression atmosphérique, par un mélange d'hydrogène et d'azote contenant 6% d'hydrogène dans l'azote entre la température ambiante et 240°C, puis sous hydrogène pur entre 240°C et 450°C.

Une solution 3 est préparée, qui contient 5,2 g de molybdène héxacarbonyle mis en solution dans 100 ml d'heptane sous atmosphère inerte.

Après refroidissement à la température ambiante sous hydrogène, le précurseur est ajouté sous inerte à la solution 3, et le mélange est maintenue sous agitation jusqu'à décoloration complète de la solution 3.

Aprés évacuation de l'hydrogène sous inerte et passivation par un mélange à 1% d'oxygène dans de l'azote, une remise à l'air progressive de la solution 3 est effectuée. Le solide est ensuite filtré, séché à l'étuve à 120°C, puis le catalyseur E ainsi obtenu est chargé dans une unité .

### Exemple 2 (selon l'invention): catalyseur F

Une solution 1 contenant 130 g d'orthosilicate d'éthyle (T.E.O.S.) dissous dans 50 ml d'éthanol et une solution 2 contenant 46,3 g de nitrate de cobalt hexahydraté, 24,15 g d'heptamolybdate d'ammonium tétrahydraté et 32 g d'acide nitrique concentré dissous dans 80 cm3 d'eau, sont mélangées sous forte agitation à température ambiante.

L'hydrolyse du T.E.O.S. conduit au bout de 19 mn à la formation d'un gel massique contenant les sels de cobalt et de molybdène.

Le gel obtenu est séparé des eaux mères, séché à l'étuve entre 40°C et 120°C, puis calciné à 600°C sous air.

Le précurseur de catalyseur ainsi obtenu est réduit, à pression atmosphérique, par un mélange d'hydrogène et d'azote contenant 6% d'hydrogène dans l'azote entre la température ambiante et 240°C, puis sous hydrogène pur entre 240°C et 450°C.

Une solution 3 est préparée, qui contient 0,21 g de trichlorure de ruthénium héxamine mis en solution dans 50 ml d'heptane sous atmosphère inerte.

Après refroidissement à la température ambiante sous hydrogène, le précurseur est ajouté sous inerte à la solution 3, et le mélange est maintenue sous agitation jusqu'à décoloration complète de la solution 3.

Après évacuation de l'hydrogène sous inerte et passivation par un mélange à 1% d'oxygène dans de l'azote, une remise à l'air progressive de la solution 3 est effectuée. Le solide obtenu est ensuite filtré, séché à l'étuve à 120°C, puis le catalyseur F ainsi obtenu est chargé dans une unité .

### Exemple 3 (selon l'invention): catalyseur G

Une solution 1 contenant 130 g d'orthosilicate d'éthyle (T.E.O.S.) dissous dans 50 ml d'éthanol et une solution 2 contenant 46,3 g de nitrate de cobalt hexahydraté, 24,15 g d'heptamolybdate d'ammonium tétrahydraté, 0,11 g de trichlorure de ruthénium héxamine, et 32 g d'acide nitrique concentré dissous dans 80 cm3 d'eau, sont mélangées sous forte agitation à température ambiante.

L'hydrolyse du T.E.O.S. conduit au bout de 19 mn à la formation d'un gel massique contenant les sels de cobalt, de molybdène et de ruthénium.

Le gel obtenu est séparé des eaux mères, séché à l'étuve entre 40°C et 120°C, puis calciné à 600°C sous air.

Le précurseur de catalyseur ainsi obtenu est réduit, à pression atmosphérique, par un mélange d'hydrogène et d'azote contenant 6% d'hydrogène dans l'azote entre la température ambiante et 240°C, puis sous hydrogène pur entre 240°C et 450°C.

Une solution 3 est préparée, qui contient 0,10 g de trichlorure de ruthénium héxamine mis en solution dans 50 ml d'heptane sous atmosphère inerte.

Après refroidissement à la température ambiante sous hydrogène, le précurseur est ajouté sous inerte à la solution 3, et le mélange est maintenue sous agitation jusqu'à décoloration complète de la solution 3.

Après évacuation de l'hydrogène sous inerte et passivation par un mélange à 1% d'oxygène dans de l'azote, une remise à l'air progressive de la solution 3 est effectuée. Le solide obtenu est ensuite filtré, séché à l'étuve à 120°C, puis le catalyseur G ainsi obtenu est chargé dans une unité .

### Exemple 4 (comparatif): catalyseur H

Une solution 1 contenant 130 g d'orthosilicate d'éthyle (T.E.O.S.) dissous dans 50 ml d'éthanol et une solution 2 contenant 46,3 g de nitrate de cobalt hexahydraté, 24,15 g d'heptamolybdate d'ammonium tétrahydraté, 0,21 g de trichlorure de ruthénium héxamine et 32 g d'acide nitrique concentré dissous dans 80 cm3 d'eau, sont mélangées sous forte agitation à température ambiante.

L'hydrolyse du T.E.O.S. conduit au bout de 17 mn à la formation d'un gel massique contenant les sels de cobalt, de molybdène et de ruthénium.

Le gel obtenu est séparé des eaux mères, séché à l'étuve entre 40°C et 120°C, puis calciné à 600°C sous air. Le catalyseur H ainsi obtenu est chargé dans une unité .

Les catalyseurs E à H préparés dans les exemples 1 à 5 comprennent donc finalement 25 % de cobalt, 5 % de molybdène et 0,18 % de ruthénium, les % étant exprimés en % poids de chaque élément par rapport au poids de silice.

### Exemple 5: Tests catalytiques

Les catalyseurs E, F, G,H dont les préparations sont décrites dans les exemples 1 à 4 ci-dessus ; sont testés en lit fixe phase gazeuse dans une unité fonctionnant en continu et opérant sur 20 cm3 de catalyseur.

Les catalyseurs E à H sont préalablement réduits in-situ jusqu'à 240°C par un mélange d'hydrogène et d'azote contenant 6% d'hydrogène dans l'azote, puis par de l'hydrogène pur jusqu'à 450°C, à la pression atmosphérique. Les conditions de test des catalyseurs sont les suivantes:
- Température 240°C
- Pression 2MPa
- Vitesse volumique horaire (V.V.H.) : 2000 h-1
- Rapport molaire H2:CO= 2:1

**TABLEAU 1**

| CONVERSION DU GAZ DE SYNTHESE EN HYDROCARBURES | | | | |
|---|---|---|---|---|
| CATALYSEUR | CONV. CO (%vol.) | DISTRIBUTION DES HYDROCARBURES FORMES (%Pds) | | |
| | | C₁ | C₂-C₄ | C₅⁺ |
| E | 58 | 7,5 | 3,8 | 89,7 |
| F | 66 | 4,8 | 3,1 | 92,1 |
| G | 72 | 6,2 | 3,5 | 90,3 |
| H | 60 | 10,5 | 4,7 | 84,8 |

## Revendications

1. Procédé de préparation d'un catalyseur comprenant un support choisi dans le groupe formé par au moins un oxyde d'élément choisi dans le groupe formé par les éléments suivants: Si, Al, Ti, Zr, Sn, Zn, Mg, Ln (où Ln est une terre rare) et, en % poids d'élément par rapport au poids de support, entre 1 et 60 % de cobalt, entre 0,01 et 20 % d'au moins un élément additionnel A choisi dans le groupe formé par le ruthénium, le platine, le palladium et l'uranium, entre 0,01 et 20 % d'au moins un élément additionnel B choisi dans le groupe formé par le molybdène et le tungstène, caractérisé en ce qu'il comprend au moins les étapes successives suivantes :
- (1) la formation d'un précurseur comprenant au moins le cobalt et au moins une partie du support
- (2) la réduction au moins partielle dudit précurseur en présence d'au moins un composé réducteur, et
- (3) le dépôt sur le précurseur réduit de toute partie de composé présent dans le catalyseur et non présent dans le précurseur.

2. Procédé selon la revendication 1 tel que le précurseur comprend en outre une partie de l'élément A présent dans le catalyseur, l'autre partie de l'élément A présent dans le catalyseur étant déposée sur le précurseur réduit à l'étape (3).

3. Procédé selon l'une des revendications 1 ou 2 tel que le précurseur comprend en outre la totalité de l'élément A présent dans le catalyseur.

4. Procédé selon l'une des revendications 1 à 3 tel que le précurseur comprend en outre une partie de l'élément B présent dans le catalyseur, l'autre partie de l'élément B présent dans le catalyseur étant déposée sur le précurseur réduit à l'étape (3).

5. Procédé selon l'une des revendications 1 ou 2 tel que le précurseur comprend en outre la totalité de l'élément B présent dans le catalyseur.

6. Procédé selon l'une des revendications 1 à 5 tel que le support est choisi dans le groupe formé par la silice, l'alumine, la zircone et l'oxyde de titane.

7. Procédé selon l'une des revendications 1 à 6 tel que le précurseur comprend la totalité du support présent dans le catalyseur.

8. Catalyseur susceptible d'être obtenu par le procédé de préparation selon l'une des revendications 1 à 7.

9. Procédé de synthèse d'hydrocarbures essentiellement linéaires et saturés, contenant au moins 80 % poids d'hydrocarbures C₅⁺ par rapport à l'ensemble des hydrocarbures formés, à partir d'un gaz de synthèse CO-(CO₂)-H₂, en présence d'un catalyseur selon la revendication 8 ou préparé selon l'une des revendications 1 à 7, tel que la conversion du gaz de synthèse en hydrocarbures est opérée sous une pression totale comprise entre 0,1 et 15 MPa, la température étant comprise entre 150 et 350 °C, la vitesse volumique horaire étant comprise entre 100 et 20 000 volumes de gaz de synthèse par volume de catalyseur et par heure, et le rapport molaire H₂/CO dans le gaz de synthèse étant compris entre 1:2 et 5:1.

10. Procédé selon la revendication 9 dans lequel le catalyseur est soumis à une préréduction avant utilisation, ladite préréduction du catalyseur étant effectuée par au moins un composé réducteur, éventuellement mis en contact avec un gaz inerte en rapport molaire composé réducteur/(composé réducteur + gaz inerte) compris entre 0,001:1 et 1:1, la préréduction étant menée entre 150°C et 600°C, de préférence entre 200 et 500°C, entre 0,1 et 10 MPa et à une vitesse volumétrique horaire de 100 à 40 000 volumes de mélange par volume de catalyseur et par heure.

## Claims

1. A process for the preparation of a catalyst comprising a support selected from the group formed by at least one oxide of an element selected from the group formed by the following elements: Si, Al, Ti, Zr, Sn, Zn, Mg or Ln (where Ln is a rare earth) and, expressed as the weight % of the element with respect to the weight of the support, 1% to 60% of cobalt, 0.01% to 20% of at least one additional element A selected from the group formed by ruthenium, platinum, palladium and uranium, and 0.01% to 20% of at least one additional element B selected from the group formed by molybdenum and tungsten, the process being characterized in that preparation of the catalyst comprises at least the following successive steps:
(1) formation of a precursor comprising at least cobalt and at least a portion of the support;
(2) at least partial reduction of the precursor in the presence of at least one reducing compound; and
(3) deposition of any part of compound present in the catalyst and not present in the precursor on the reduced precursor.

2. A process according to claim 1, in which the precursor further comprises a portion of the element A present in the catalyst, the other portion of element A present in the catalyst being deposited on the reduced precursor in step (3).

3. A process according to claim 1 or claim 2, in which the precursor further comprises the totality of element A present in the catalyst.

4. A process according to any one of claims 1 to 3, in which the precursor further comprises a portion of element B present in the catalyst, the other portion of element B present in the catalyst being deposited on the reduced precursor in step (3).

5. A process according to claim 1 or claim 2, in which the precursor further comprises the totality of element B present in the catalyst.

6. A process according to any one of claims 1 to 5, in which the support is selected from the group formed by silica, alumina, zirconia and titanium oxide.

7. A process according to any one of claims 1 to 6, in which the precursor further comprises the totality of the support present in the catalyst.

8. A catalyst prepared using the preparation process defined in any one of claims 1 to 7.

9. A process for the synthesis of essentially linear saturated hydrocarbons containing at least 80% by weight of C₅⁺ hydrocarbons with respect to the totality of the hydrocarbons formed, from a synthesis gas CO-(CO₂)-H₂, in the presence of a catalyst according to claim 8 or prepared in accordance with any one of claims 1 to 7, in which conversion of the synthesis gas to hydrocarbons is carried out at a total pressure in the range 0.1 to 15 MPa, the temperature is in the range 150°C to 350°C, the hourly space velocity is in the range 100 to 20000 volumes of synthesis gas per volume of catalyst per hour, and the H₂/CO molar ratio in the synthesis gas is in the range 1:2 to 5:1.

10. A process according to claim 9, in which the catalyst is pre-reduced before use, said pre-reduction being carried out using at least one reducing compound, which is optionally brought into contact with an inert gas in a reducing compound/(reducing compound + inert gas) molar ratio in the range 0.001:1 to 1:1, pre-reduction being carried out between 150°C and 600°C, preferably between 200°C and 500°C, between 0.1 and 10 MPa and at an hourly space velocity of 100 to 40000 volumes of mixture per volume of catalyst per hour.

## Patentansprüche

1. Verfahren zur Herstellung eines Katalysators, einen Träger umfassend, der aus der Gruppe gewählt ist, die gebildet ist durch Wenigstens ein Oxid eines Elementes, gewählt aus der Gruppe, die durch die folgenden Elemente gebildet ist: Si, Al, Ti, Zr, Sn, Zn, Mg, Ln (wo Ln eine seltene Erde ist) und in Gew.-% des Elementes bezogen auf das Gewicht des Trägers zwischen 1 und 60% Kobalt, zwischen 0,01 und 20% wenigstens eines Zusatzelementes A, gewählt aus der Gruppe, die gebildet ist durch Ruthenium, Platin, Palladium und Uran, zwischen 0,01 und 20% wenigstens eines Zusatzelementes B, gewählt aus der Gruppe, die gebildet ist durch Molybdän und Wolfram, dadurch gekennzeichnet, daß es wenigstens die folgenden Stufen umfaßt:
- (1) die Bildung eines Vorläufers, der wenigstens Kobalt und Wenigstens einen Teil des Trägers umfaßt
- (2) die wenigstens partielle Reduktion des Vorläufers in Anwesenheit wenigstens einer reduzierenden Verbindung und
- (3) die Abscheidung auf dem reduzierten Vorläufer des gesamten Teils der Verbindung, die im Katalysator und nicht im Vorläufer vorhanden ist.

2. Verfahren nach Anspruch 1, derart, daß der Vorläufer im übrigen einen Teil des im Katalysator vorhandenen Elementes A umfaßt, wobei der andere Teil des im Katalysator vorhandene Elementes A auf dem in Stufe (3) reduzierten Vorläufer abgeschieden wird.

3. Verfahren nach Anspruch 1 oder 2, derart, daß der Vorläufer im übrigen die Gesamtheit des im Katalysator vorhandenen Elementes A umfaßt.

4. Verfahren nach einem der Ansprüche 1 bis 3, derart, daß der Vorläufer im übrigen einen Teil des im Katalysator vorhandenen Elementes B umfaßt, wobei der andere Teil des im Katalysator vorhandenen Elementes B auf dem in Stufe (3) reduzierten Vorläufer abgeschieden wird.

5. Verfahren nach einem der Ansprüche 1 oder 2, derart, daß der Vorläufer im übrigen die Gesamtheit des im Katalysator vorhandenen Elementes B umfaßt.

6. Verfahren nach einem der Ansprüche 1 bis 5, derart, daß der Träger aus der durch Siliziumoxid, Aluminiumoxid, Zirkon und Titanoxid gebildeten Gruppe gewählt ist.

7. Verfahren nach einem der Ansprüche 1 bis 6, derart, daß der Vorläufer im übrigen die Gesamtheit des im Katalysator vorhandenen Trägers umfaßt.

8. Katalysator erhältlich nach dem Herstellungsverfahren eines der Ansprüche 1 bis 7.

9. Verfahren zur Synthese von im wesentlichen linearen und gesättigten Kohlenwasserstoffen, die wenigstens 80 Gew.-% C₅⁺ Kohlenwasserstoffe bezogen auf die Gesamtheit der gebildeten Kohlenwasserstoffe enthalten, ausgehend von einem Synthesegas CO-(CO₂)-H₂, in Anwesenheit eines Katalysators gemäß Anspruch 8 oder hergestellt nach einem der Ansprüche 1 bis 7, derart, daß die Umwandlung des Synthesegases in Kohlenwasserstoffe unter einem Gesamtdruck zwischen 0,1 und 15 MPa durchgeführt wird, wobei die Temperatur zwischen 150 und 350°C, die stündliche Volumengeschwindigkeit zwischen 100 und 20 000 Volumenteile Synthesegas pro Volumenteile Katalysator und Stunde und das Molverhältnis H₂/CO im Synthesegas zwischen 1:2 und 5:1 liegt.

10. Verfahren nach Anspruch 9, bei dem der Katalysator einer Vorreduktion vor seiner Verwendung ausgesetzt wird, wobei die Vorreduktion des Katalysators durch wenigstens eine reduzierende Verbindung durchgeführt wird, die gegebenenfalls mit einem inerten Gas in einem Molverhältnis reduzierende Verbindung/(reduzierende Verbindung + inertes Gas) zwischen 0,001:1 und 1:1 kontaktiert wird und die Vorreduktion zwischen 150°C und 600°C, bevorzugt zwischen 200 und 500°C, zwischen 0,1 und 10 MPa und bei einer stündlichen Volumengeschwindigkeit von 100 bis 40 000 Volumenteile Gemisch pro Volumenteil Katalysator und Stunde durchgeführt wird.
